# EUROPEAN PATENT SPECIFICATION

(11) **EP 1 149 078 B1**
(45) Date of publication and mention of the grant of the patent: **08.03.2006**
(21) Application number: 99967248.8
(22) Date of filing: 10.12.1999
(51) Int. Cl.: C07D 209/04, C07D 209/60, A01N 43/38

(54) **SELECTIVE 5-HT 6 RECEPTOR LIGANDS**
SELEKTIVE 5-HT 6-REZEPTOR-LIGANDEN
LIGANDS DU RECEPTEUR DE 5-HT 6 SELECTIFS

(30) Priority: 11.12.1998 US 111787 P
(43) Date of publication of application: 31.10.2001
(73) Proprietor: VIRGINIA COMMONWEALTH UNIVERSITY, Richmond, VA 23298 (US)
(72) Inventor: GLENNON, Richard, A., Midiothian, VA 23113 (US); ROTH, Bryan, L., Morelandhills, OH 44022 (US)
(74) Representative: Dolan, Anthony Patrick
(86) International application number: PCT/US1999/029219
(87) International publication number: WO 2000/034242

(56) References cited:
- US-A- 3 939 177
- US-A- 4 853 391
- GLENNON ET AL.: 'Influence of Amine Substituents on 5-HT2A versus 5-HT2C Binding of Phenylalkyl- and Indolylalkylamines' J. MED. CHEM., vol. 37, no. 13, 1994, pages 1929 - 1935, XP002927192

## Description

This invention was discovered in the performance of U.S.govemment supported research under grants NIMH KO2MH01366 and NCEMS GM52213, and the U.S. government may have certain rights in the invention.

### DESCRIPTION

### BACKGROUND OF THE INVENTION

### Field of the invention

The present invention relates to the synthesis of novel ligands selective for a subgroup of receptors for serotonin (5-HT). While there are seven subgroups of 5-HT receptors, this invention is selective for the 5-HT₆ subgroup. This invention also relates to the synthesis of novel ligands selective for the 5-HT₆ subgroup receptor that act as agonists to the natural ligands for this receptor. The invention also relates to the creation of novel ligands that act as antagonists to the 5-HT₆ receptor. The invention further relates to use of said compounds to treat mammals adversely affected by conditions mediated by the 5-HT₆ receptor.

### Description of the prior art

Serotonin receptors have been divided into a number of families and subfamilies (5-HT₁-5-HT₇) and approximately 14 populations have been cloned. One of the newest populations identified is the 5-HT₆ subgroup. It has been observed that various tricyclic psychotropic agents (neuroleptics, atltidepressants, and atypical neuroleptics agents) bind the 5-HT₆ receptor with nanomolar affinities (Roth et al. J. Pharmacol. Exp. Ther. 1994, 268, 1403-1410). A rat 5-HT₆ receptor was first cloned in 1993 and, more recently, the same group described the cloning of a human 5-HT₆ receptor. The 5-HT₆ serotonin receptors are members of the G-protein superfamily, are positively coupled to an adenylate cyclase second messenger system, and are found primarily in the central nervous system. Serotonin bound to the 5-HT₆ receptor subgroup causes an activation of the adenylate cyclase enzyme, with concomitant increased levels of intracellular cAMP. Although the exact physiological function and clinical significance of the 5-HT₆ receptor subgroup is not known, as noted above, many anti- psychotic agents bind these receptors with high affinity. Also, in rats that do not express 5-HT₆ receptors, the animals behave in a manner that seems to involve an increase in cholinergic function, suggesting that 5-HT₆ specific ligands might be of value in the treatment of anxiety-related disorders and memory deficits.

Upon binding to cellular receptors, ligands may act as agonists or antagonists to endogenous receptor-ligand function. In the case of the 5-HT₆ receptor, several specific ligands have been discovered which act as 5-HT₆ specific antagonists, but prior to the present invention, selective ligands which act as agonists to the 5-HT₆ receptor were unknown.

### SUMMARY OF THE INVENTION

It is an object of the invention to create derivatives of serotonin (5-HT) that specifically bind the 5-HT₆ receptor subgroup of the serotonin receptor family. It is another object of this invention to create 5-HT₆- selective ligands that act as agonists when bound to the 5-HT₆ receptor. It is further an object of this invention to create 5-HT₆-selective ligands that act as antagonists when bound to the 5-HT₆ receptor. Furthermore, the compounds of the present invention that possess antagonist activity are tryptamine derivatives and are structurally unrelated to previously described 5-HT₆ antagonists. It is further an object of this invention to administer 5-HT₆ selective ligands to animals to determine the physiological and biochemical effects of specific activation and inhibition of 5-HT₆ receptor function. Finally, it is an object of this invention to treat mental disorders mediated by 5-HT₆ function by administering to treatment subjects the 5-HT₆-selective agonists and antagonist compounds described herein.

Various indolealkylamines, including serotonin(5-HT) and 5-methoxytryptamine, have been observed to bind the 5-HT₆ receptor with high affinity and produce a potent dose-dependent increase in cAMP levels. These tryptamines, however, are non-selective and bind at multiple families of 5-HT receptors. According to the invention, various modifications of 5-HT have been made to generate ligands with selectivity for the 5-HT₆ receptor. An analog of 5-HT with a 2-methyl substituent introduced (2-methyly-5-HT) binds the 5-HT₆ receptor with an affinity equivalent to that of the parent compound. The above analog is selective for the 5-HT₆ and 5-HT₃ receptors and binds at the 5-HT₆ subgroup with a 20 fold greater affinity than at 5-HT₃ receptors.

A 2-methyl analog of 5-methoxytryptamine, 5-methoxy-2-methyltryptamine, binds to the 5-HT₆ receptor with an affinity comparable to 2-methyl-5-HT. However, 5-methoxy-2-methyltryptamine lacks affinity for 5-HT₃ receptors. Thus, 5-methoxy -2-methyltryptamine presents a ligand with specificity for the 5-HT₆ receptor subgroup. In the present invention, the 5-methoxy-2-methyltryptamine compound has been modified and several of its alkyl derivatives bind with comparable affinity and activate adenylate cyclase activity at levels comparable to serotonin. Furthermore, one derivative, 5-methoxy-2- phenylotryptamine, binds to the 5-HT₆ receptor with a high affinity but the phenyl addition renders the compound an antagonist to 5-HT stimulated adenylate cyclase activity.

### BRIEF DESCRIPTION OF THE DRAWINGS

The foregoing and other objects, aspects and advantages will be better understood from the following detailed description of the preferred embodiments of the invention with reference to the drawings, in which:

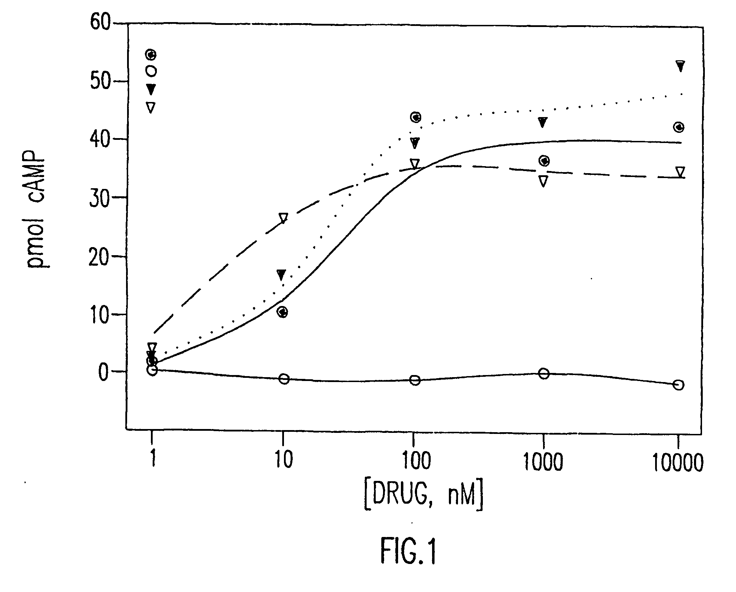

Figure 1 is a graph showing the adenylate cyclase activity observed with several compounds of the invention.

### DETAILED DESCRIPTION OF THE PREFERRED EMBODIMENTS OF THE INVENTION

The invention provides novel tryptamine derivative compounds with selectivity for the 5-HT₆ receptor subgroup. For the present purpose, an agent is termed selective when it displays an affinity for 5-HT₆ receptors that is tenfold higher than affinities it displays for other related receptor populations. The invention further provides a method using said compounds to receptor subtype 5-HT₆ as agonists, or as antagonists to serotonin. The compounds of the invention can be used either as the free base or as the pharmaceutically acceptable acid-addition salt form, for example, hydrochloride, hydrobromide, tartrate, and maleate. They may be used in oral or injectable pharmaceutical preparations as prophylactic and acute-phase remedies for the relief and reversal of serotonin-regulated symptoms. They may be used alone or in combination with each other or other known medications. Finally, said compounds may be used as above for determining 5-HT₆ receptor function.

Serotonin (5-hydroxy tryptamine, or 5-HT) is a product of tryptophan metabolism and is a tryptamine derivative that is a potent neurotransmitter. Serotonin is a well-characterized tryptamine derivative which regulates calcium ion channels on the surface of nerve and muscle cells. Many mental disorders in humans are associated with fluctuations in serotonin levels and are effectively treated with drugs which specifically interact with serotonin receptors or that block the reuptake of serotonin into the presynaptic axon terminals, suggesting that serotonin dysregulation may be involved in various mental disorders. Some serotonin receptor ligands and are clinically approved as drugs for the treatment of migraine headaches, depression, high blood pressure, and psychosis.

Generally, tryptamine derivatives are non-selective and bind at multiple 5-HT receptor subgroups. Serotonin is no exception and binds at the various subfamilies of the 5-HT receptor, including the 5-HT₆ subgroup where it is a potent activator of adenylate cyclase enzyme activity. Serotonin has the chemical formula:

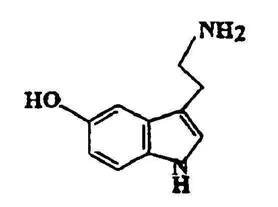

Some modifications of serotonin result in loss of affinity for various 5-HT receptor subgroups. It had previously been thought that introduction of a 2-methyl substituent to 5-HT was not tolerated by any 5-HT receptors but the 5-HT₃ subgroup. Thus, prior to identification of the 5-HT₆ receptor, 2- methylation of 5-HT was thought to render the product selective for the 5-HT₃ subgroup. We have found that the two methyl derivative of 5-HT, 2-methyl-5-HT has a high affinity for the 5-HT₆-receptor. In fact, 2-methyl-5-HT binds the 5-HT₆ receptor with a 20 fold greater affinity over 5-HT₃ receptors. The 5-HT₆-selective ligand 2-methyl-5-methoxytryptamine contains a primary amine, presenting an obstacle to the compound crossing the blood brain barrier and also rendering the compound vulnerable to rapid metabolism due to oxidative deamination. Our discovery that a methyl substituent at the 2 position was tolerated by the 5-HT₆ receptor, together with the previous observation that O-° methylation of 5-HT abolishes affinity for 5-HT, receptor, led to the present invention.

To address the above limitations of 2-methyl-5-methoxytryptamine, several derivative compounds were synthesized that were lipophilic and also might be less prone to rapid metabolism. N,N-dimethyl substituents were added to 2-methyltryptamine to create 2-methyl-N,N-dimethylayptamine (Ki=308nM). Re-introduction of the methoxy group to this compound, to form 2-methyl-5-methoxy-N,N-dimethyltryptamine resulted in a compound (Compound A) with an affinity for the 5-HT₆ receptor of Ki=60nM. Homologation of the 2-methyl substituent of the above compound to form 2-ethyl-5-methoxy-N,N-dimethyltryptamine resulted in a ligand with an increased affinity for the 5-HT₆ of Ki=16nM (Compound B). To determine whether or not greater bulk additions could be added in place of a methyl or ethyl group, the 2-phenol derivative of the 2-methyl-5-methoxy-N,N-dimethyltryptamine was generated. This compound D binds the 5-HT₆ receptor with a Ki=20nM. These derivatives were of the general formula 1 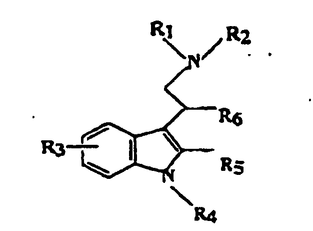
where R₁ and R₂=H or CH₃,
R₅=H, OH, OCH₃, or a substituted or unsubstituted alkyl,
R₄=H, CH₂-phenyl, SO₂-phenyl, or CH₂ as part of a substituted or unsubstituted alkyl ring connecting R₄ and R₅,
R₅=H, CH₃, or CH₂ as part of a substituted or unsubstituted alkyl ring connecting R₅ with either R₄ or R₆,
R₆=H, CH₃, or CH₂ as part of a substituted or unsubstituted alkyl ring connecting R₆ and R₅. Thus, the present invention relates to the compounds as defined in any one of claims 1 to 6 or 9.

The compounds of the invention and the pharmaceutically acceptable salts of the compounds of the invention can be used in the form of pharmaceutical preparations. The preparations can be administered orally, for example in the form of tablets, coated tablets, dragees, hard and soft gelatin capsules, solutions, emulsions or suspensions. The administration can be effected rectally, for example in the form of suppositories, or parenterally, for example in the form of injection solutions.

The compounds of the invention can be processed with pharmaceutically inert, inorganic or organic carriers for the production of pharmaceutical and research preparations. The preparations can contain preservatives, solubilizers, wetting agents, emulsifiers, sweeteners, colorants, flavorants, salts for varying the osmotic pressure, buffers, masking agents or antioxidants. They can also contain still other therapeutically valuable substances.

The compounds of the present invention can also be radiolabelled and used to identify other 5-HT₆ ligands using techniques common in the art. This can be achieved by incubating the receptor in the presence of a ligand candidate plus an equimolar amount of radiolabelled compound of the invention. Ligands selective for 5-HT₆ are then revealed as those that are not significantly displaced by the compounds of the present invention.

Another embodiment of the invention can be the administration of the compounds of the invention to animals in drug discrimination assays. In a drug discrimination paradigm, animals (usually rats) can be trained to recognize the effects of a given agent. Once trained, these animals can be used in tests of stimulus generalization to identify other agents that produce similar effects (i.e., agonists), or the animals can be used in tests of stimulus antagonism to identify agents that block or antagonize (i.e., antagonists) the effects of the training drug. Hence, the procedure can be used to identify agonists that produce an effect common to the training drug, more antagonists that can block the effects of the training drug. Specifically, with a 5-HT₆-selective agonist as training drug, the animal can be used to identify other 5-HT₆ agonists and to identify 5-HT₆-antagonists.

One family of compounds contemplated for use in this invention is represented by the formula 2 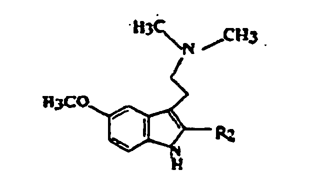
wherein R₂ is selected from the group consisting of ethyl, n-propyl, and phenyl.

Another family of compounds for use in this invention is represented by formula 3 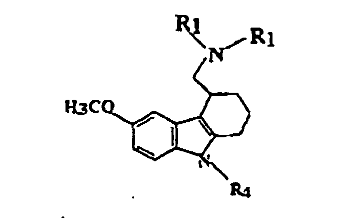
wherein R₁ is selected from the group consisting of lower alkyls such as ethyl and propyl, methyl and hydrogen, and can be the same or different at each location, and R₄ is from the group comprising H, methyl, ethyl or propyl.

The following examples illustrate the present invention in more detail.

### Example 1

Synthesis of 2-ethyl-5-methoxy-N,N-dimethyltryptamine maleate (Compound B). A 2.5M solution of nBuLi (1.75 ml. 4.38 mmol) was added in a drop wise manner to a stirred solution of 2-methyl-5-methoxy-N,N-dimethyltryptamine (compound A in the examples below) free base (1.00 g, 4.33 mmol) in dry THF (7 mL) at -78 °C under N₂.

After stirring the reaction mixture for five minutes, the cooling bath was removed and CO₂ gas was passed into the solution for 10 minutes. The solvent was removed at 0 °C under reduced pressure to give a transparent solid. The flask was flushed with N₂ and dry THF (7 mL) was added. The reaction mixture was degassed at -150 °C under reduced pressure of 1mMHg, then allowed to warm to -78 °C; 1.7M tNuLi (2.8 mL, 4.8 mmol) was added in a drop wise manner. The solution was kept at -78 °C for three hours. The reaction make sure was acidified with a saturated ethereal solution of HCl. Anhydrous Et₂O was added to the resulting suspension and the supernatant was decanted. The residue was heated at 100 °C under reduced pressure for 20 minutes. The resulting residue was purified by flash chromatography on silica gel (CH₂Cl₂/MeOH; 12:1) to give 0.17 g of a bright yellow oil (16%). ¹H-NMR(CDCl₃) d 8.06 (s, 1H, J=8.67 HZ), 6.98 (s, 1H), 6.76 (dd, 1H, J=2.34, 8.73 HZ), 3.84 (s, 3H) 2.91-2.87 (m, 2H), 2.71 (q, 2H, J=7.38 HZ), 2.57-2.52 (m, 2H) 2.38 (s, 6HJ)1.25 (t, 3H, J=7.38 HZ).The maleate salt was prepared and recrystallized from an EtOAc/Et₂O mixture; mp 123 °C.

### Example 2

Magnesium turnings and NH₄Cl were added to a solution of Example 11 *infra* (1-Benzenesulfonyl-5-methoxy-2-n-propyl-N,N-dimethyl tryptamine, free base; 259 mg, 0.65 mmol) in MeOH (17 mL) and the mixture was allowed to stir at room temperature for one-hour. Saturated NH₄Cl solution was added and the reaction mixture was extracted with CH₂Cl₂. The organic portion was dried (MgSO₄) and the solvents was removed under reduced pressure. The residue was purified by flash chromatography on silica gel (CH₂Cl₂/MeOH; 9: 1) to give 75 mg of a bright yellow oil. ¹H-NMR (CDCl₃) 0 7.71 (brs, 1H), 2.89-2.83 (m, 2H), 2.69 (t, 2H, J=7.56 Hz), 2.53-2.47 (m, 2H), 2.36 (s, 6H), 1.68 (tq, 2H, J=7.28, 7.56 HZ), 0.98 (t, 3H,J=7.28 HZ). The salt was prepared and recrystallized from acetone; mp 146-147 °C.

### Example 3

5-methoxy-2-pbenyl-N,N-dimethyltryptamine oxalate (Compound D). 5-methoxy-2-phenylindole (3 g, 13.44 mmol) was added to a stirred ice-cooled solution of 1-dimethylamino-2-nitroethylene (1.56 g, 13.44 mmol) in trifluoracetic acid (8ml). The resulting mixture was allowed to stir under N₂ at room temperature for 30 minutes and was then poured into ice/water. The solution was extracted with EtOAc and the organic portion was washed consecutively with saturated NaHCO₃ solution, H₂O, and then brine. The organic portion was dried (MgSO₄) and solvent was removed under reduced pressure. The residue was recrystallized from CH₂Cl₂/hexane to give 2.36 g (60%) of a red powder. ¹H-NMR (acetone-d6) d 8.82 (brs, 1H), 3.92 (s, 3H), 1R (Kbr)1601, 1475, 1251 cm⁻¹. A solution of this material (2 g, 6.75 mmol) in dry THF was added in a drop wise manner to a cooled 0 °C suspension of LiALH₄ (1.54 g, 40.5 mmol)in dry THF under N₂. The reaction mixture was heated at reflux for one-hour and then allowed to stand at room temperature overnight. The resulting mixture was quenched with H₂O then 15% NaOH solution. Celite was added and the solution was filtered. The solvent was removed under reduced pressure. The residue was purified by flash chromatography on silica gel (CH₂Cl₂/MeOH; 9:1) to give 1 g (55 %) of the primary amine as an oil. ¹H-NMR (CDCl₃) d 8.19 (brs, 1H, J=2.37 HZ), 6.88 (dd, 1H, J=2.24, 8.75 HZ), 3.89 (s, 3H), 3.04 (brs, 4H). IR(KBr) 3397, 3347 cm⁻¹. Sodium cyanoborohydride (510 mg, 8.12 mmol) was added to a solution of the primary amine (700 mg, 2.63 mmol) and 37% aqueous CH₂O in MeCN (10 mL) at room temperature. The resulting mixture was adjusted to pH 5 with HOAc and was allowed to stir at room temperature overnight. A 15% solution of NaOH was added to neutralize the mixture and the mixture was extracted with. CH₂Cl₂. The combined organic portion was washed with saturated NaHCO₃ solution and brine. The organic portion was dried (MgSO₄)and solvent was removed under reduced pressure. The residue was purified by flash chromatography on silica gel (CH₂Cl₂/MeOH; 9: 1) to give 195 mg of 5-methoxy-2-phenyl-dimethyltryptamine free base as a white powder. ¹HNMR (CDCl₃) d 8.05 (brs, 1 h), 7.56-7.53 (m, 2H),7.49-7.44 (m, 2H), 7.39-7.34 (m, 1H), 7.29, 7.25 (m, 1H), 7.11 (d, 1H, J=2.25 HZ). 6.87 (dd, 1H, J=2.52, 8.73 HZ), 3.89 (s, 3H). 3.13-3.08 (m, 2H), 2.72-2.66 (m, 2H), 2.39 (s, 6H). Although the HCl salt has previously been reported, difficulties in his purification led to isolation of the product has its salt; after recrystallization from acetone.

### Reference

### Example 4

4-dimethylaminomethyl-9-benzyl-6-methoxy-1,2,3,4-tetrahydrocarbazole hydrochloride (Compound E). A mixture of 4-methoxyphenyl)benzylamine (42 g 0.2 mol) and ethyl 6-bromocyclohexanone Carboxylate (J. Org Chem 1961, 26, 22) (24.9 g, 0.1 mol) were heated at reflux in dry benzene (250 mL) for 24 hours. The reaction mixture was cooled and precipitated (4-methoxyphenyl) benzylamine hydrobromide was separated by filtration. The benzene extract was concentrated and fused zinc chloride (40 g) was added in reflux in absolute ethanol (125 mL) for six hours. The cooled mixture was slurred in H₂O (250 mL) and extracted with (Et₂O; 4x 200 mL). The combined ether extracts were washed with 5% HCl (100 mL), followed by brine solution and dried with MgSO₄. The ether extract was evaporated under reduced pressure to give crude ethylester of title compound which was treated with a solution of KOH (50 g) in H₂O (150 mL) and CH₃OH (150 mL) at reflux temperature for three hours. The solution was evaporated to dryness under reduced pressure, the resulting residue was dissolved in H₂O (250 mL) and the aqueous solution was extracted with Et₂O and acidified with 10 % HCl. The resulting solid was dried to give 22 g (33%) of title compound and was recrystallized from ls.PrOH-H2). Mp 212-214 °C. To a mixture of sodium hydride (0.96 g, 0.04 mol) in dry benzene (200 mL) was added portion wise 9-benzyl-6-methoxy-1,2,3,4-tetrahydro-4-carboxylic acid (14.00 g, 0.04 mol) and the mixture was stirred for one-hour. Thionoyl chloride (3.00 mL, 0.04 mol) was added slowly in the stirring was continued for 30 minutes. The resulting solution was poured into aqueous dimethylamine solution (40 %)(36.50 mL) with ice bath cooling. The mixture was third for one-hour, washed with 100 mL H₂O, NaHCO₃ (50 mL)and saturated brine solution (50 mL), and dried with MgSO₄, diluted with n-pentane (200 mL)and cooled to give 4-dimethylaminocarbonyl-9-benzyl-6-methoxy-1,2,3,4-tetrahydrocarbazole (9.10 g, 60 %) mp 153-155 °C. To a stir it solution of LialH₄ (4.71 g, 94.2 mmol) in dry THF was added portion wise 4-dimethylaminocarbonyl-9-benzyl-6-methoxy-1,2,3,4-tetrahydrocarbazole (9.00 g, 24.8 mmol) and the mixture was heated under reflux for five hours the reaction mixture was cooled water (5.0 mL) NaOH solution (5.0 mL) was added and filtered. The filtrate was evaporated to dryness to give 4-dimethylamino-9-benzyl-6-methoxy-1,2,3,4-tetrahydrocarbazole (8g, 92 %). The free base was dissolved in either in converted to the salt using ethereal hydrochloride and recrystallized from a mixture of EtOH and Et₂O, Mp 238-240 °C.

### Reference

### Example 5.

Compound F is known and was prepared according to the following patent procedure:4-aminomethyl-9-benzyl-1,2,3,4-tetrahydrocarbazoles, U.S. patent No. 3,939,177. Feb 17, 1976.

### Example 6

Compound G Sodium metal was added portion wise over a thirty minute period to a stirred solution of 4-(dimethylaminomethyl)-9-benzyl-6-methoxy-1,2,3,4-tetrahydrocarbazole (4 g, 0.01 mol) in liquid NH₃ (300 mL). NH₄Cl (3.0 g) was added until the blue-collar of the mixture dissipated. The NH₃ was evaporated, water (50 mL) was added in the mixture was extracted withCH₂CL₃. The combined organic portion was washed with water (50 mL), brine (50 mL), dried (MgSO₄) and evaporated to give an oil. The oil was purified by column chromatography (CHCL₃/MeOH; 9:1) and converted to an oxalate salt. The salt was recrystallized from anhydrousEt₂O/absolute-EtOH to give 1.8 g of the desired target as a white powder.¹H-NMR (CDCl₃, free base) d 8.10 (s, 1H, NH), 7.20 (t, 1H, ArH), 6.90 (d, 1H, ArH), 6,70 (dd, 1H, ArH), 3.80 (s, 3H, OCH3), 3.40 (t, 1H, CH), 3.15 (d, 1H, CH), 3.0 (t, 1H, CH)3.00 (t, 1H, Ch), 2.82 (s, 6H, 2XCH3), 2.63-2.73 (m, 2H, CH2), 2.23 (m, 1H, CH) 1.8-2.0 (m, 3H, CH2-CH).

### Example 7

A mixture of Compound G as free base (.5 g, 1.94 mmol) and sodium hydride (60%) (0.085 g, 3.54 mmol) was heated at 100 °C under nitrogen until the evolution of H₂ gas ceased. The resultant was dissolved in anhydrous DMF and benzenesulfonylchloride (0.30 mL, 2.35 mmol) was added drop wise at 0 °C.. The reaction mixture was stirred at room temperature overnight. Saturated NaHCO₃ solution was added and extracted with CH2Cl₂ (3 x 25 mL). The organic layer was dried over MgSO₄ and the solvent was removed under pressure. The residue was purified by column chromatography (CH₂Cl₂/MeOH; 9:1) as eluent to give an oil (0.60 g, 76 %) and converted to hydrochloride salt. The hydrochloride salt was recrystallized from ethanol and anhydrous ether mp 259-261 °C.

### Reference

### EXAMPLE 8

6,7,8,9-tetrahydro-2-methoxy-10-(N,N-dimethylaminoethyl)pyridol[1,2-a]indole-9-one Oxalate (I). A mixture of 5-methoxy-N,N-dimethyltryptamine (free base) (2.00 g, 9.17mmol) and 60% NaH (0.41 g, 10.1mmol) was heated at 100 °C under N₂ until evolution of gas had ceased. The resultant mass was dissolved in anhydrous DMF (25 ml) and anhydrous g-butryolactone 1.4 mL,18.2 mmol) was added in dropwise manner at room temperature. The reaction mix was heated at reflux for 20 h, cooled to 0 °C, and acidified by the addition of an ethereal solution of HCl. Additional Et₂O was added to the resulting suspension and the supernatant was decanted The residue was dissolved in PPE (52.5 mL) and CHCl₃ (100 mL) and the reaction mixture was heated at reflux for 3 h under N₂. The resulting mixture was neutralized by the addition of 15% NaOH solution at ice-bath temperature, and extracted with CH₂Cl₂. The organic portion was dried (MgSO₄) and solvent was removed under reduced pressure. The residue was purified by flash chromatography on silica gel (CH₂Cl₂/MeOH; 20:1) to give 0.52 g (20%) of 6,7,8,9-tetrahydro-2-methoxy-10-(N,N-dimethylaminoethyl)pyridol[1,2-a]indole-9-0ne free base as a yellow oil. ¹H-NMR (DMSO-d₆) □ 7.35 (d, 1H, J=8.79 Hz) 7.18 (s, 1H), 6. 88 (d, 1H, J=8.85 Hz), 4.06 (t, 2H, J=6.60 Hz), 3.80 (s, 3H), 3.42-3.36 (m, 2H), 3.17-3.12 (m, 2H), 2.85 (s, 6H), 2.66-2.62 (m, 2H0, IR (CHCl₃) 1648 ^{m-1}. A small sample was converted to the oxalate salt; mp 191-192 °C.

### Reference

### EXAMPLE 9

6,7,8,9-tetrahydro-2-methoxy-10-(N,N-dimethylaminoethyl)pyridol[1,2-a]indole Oxalate (J). A solution of 1.0 M borane/THF (2 mL, 2mmol) was added ion a dropwise manner to ice-bath cooled 6,7,8,9-tetrahydro-2-methoxy-10-(N,N-dimethylaminoethyl)pyridol[1,2-a]indole-9-one Oxalate (290 mg, 1.01 mmol) under N₂. The reaction mixture was allowed to stir at room temperature for 2 h. Acetone (3 ml) was added , and the reaction mixture was heated at reflux for 1 h to quench the unreacted borane reagent. The solvent was removed under reduced pressure. The residue was purified by flash chromatography on silica gel (hexane/EtOAc; 4:1) to give 207 mg (75%) of a light yellow oil. ¹H-NMR (DMSO-d₆) □ 7.34 (d, 1H, J=8.85 Hz) 7.21 (s, 1H), 4.08 (t, 2H, J=6.65 Hz), 3.79 (s, 3H), 3.40-3.35 (m, 2H), 3.30-3.25 (m, 2H), 3.06-3.01 (m, 2H) 2.83 (s, 6H) 1.76-1.69 (m, 2H), 1.40-1.31 (m, 2H). A small portion was converted to its oxalate salt; mp 114-115 °C.

### Reference

### EXAMPLE 10

1-Benzenesulfonyl-5-methoxy-N,N,-dimethyltryptamine oxalate. A mixture of 5-methoxy-N,N-dimethyltryptamine (free base) (4.35g, 19.93 mmol) and 60% NaH (0.87 g, 21.75 mmol) was heated at 100 °C under N₂ until evolution of H₂ gas ceased. The resultant mass was dissolved in anhydrous DMF (21 ml) and benzenesulfonyl chloride (2.8 ml, 21.94 mmol) and 60% NaH (0.87 g, 21.75 mmol) was added in a dropwise manner at 0°C. The reaction mixture was allowed to stir at room temperature overnight. Saturated NaHCO₃ solution was added and the mixture was extracted with CH2Cl2. The organic portion was dried (MgSO₄)and the solvent was removed under reduced pressure. The residue was purified by flash chromatography on silica gel (CH₂Cl₂/MeOH; 9:1) to give 4.39 g of an oil (61%). ¹H-NMR (CDCl₃) □ 7.89-7.87(m, 1H,) 7.83 (d, 2H, J=8.0 Hz), 7.51 (t, 1H, J=7.8 Hz), 7.34 (s, 1H), 6.93-6.92 (m, 2H), 3.82 (s, 3H), 2.80 (t, 2H, J=7.8 Hz) 2.59 (t, 2H, J=7.8 Hz), 2.33 (s, 6H). IR CHCl₃, 1357, 1115 c^{m-1}. The oxalate salt was prepared and recrystallized from an acetone/Et2O mixture; mp 224-226 °C.

### Reference

### EXAMPLE 11

1-Benzenesulfonyl-5-methoxy-2-n-propyl-N,N-dimethyl tryptamine. A 2.5 M solution of nBuLi (1.4mL, 3.5 mmol) was added in a dropwise manner to a stirred solution of 1-Benzenesulfonyl-5-methoxy-N,N,-dimethyltryptamine oxalate (free base) (1.00g, 2.79mmol) in DME (4 mL) at -10 °C under N₂. The resulting solution was allowed to stir for an additional 10 min at -10 °C, and then nPrl (0.35 mL, 3.59mmll) was added. The reaction mixture was allowed to stir for 1 h at -10 °C. Saturated NaHCO₃ solution was added and the reaction mixture was extracted with CHCl2. The organic portion was washed with brine and dried (MgSO₄); the solvent was removed under reduced pressure and the residue was purified by flash chromatography on silica gel. (CH₂Cl₂/MeOH;30:1). To give 0.19 g ofa bright yellow oil. ¹H-NMR (CDCl₃) □ 8.06 (d, 1H, J=8.79 Hz), 7.62 (d, 2H, J=8.22Hz), 7.51-7.46 (m, 1H), 6.89-6.85 (m, 1H), 3.85 (s, 3H), 2.96-2.89 (m, 4H) 2.63-2.57 (m, 2H), 2.48 (s, 6H) 6.87, 1.73 (q, 2H, J=7.51 Hz), 1.00 (t, 3H, J=7.51Hz). IR CHCl₃, 1355 c^{m-1}.

### EXAMPLE 12

### 5-HT derivative binding to 5-HT₆ receptor

The binding assay employed human 5-HT₆ stably transfected to HEK 293 human embryonic kidney cells with [³H] lysergic acid diethylamide (70Ci/mmol; Dupont NEN) as radioligand. Radioligand was diluted in incubation buffer in borosilicate glass vials and protected from light. Competing agents (1mM stock solution) were dissolved in DMSO or saline and stored at -20 0 C. Dilutions of compounds were made using incubation buffer in 96-well plates and mixed by multichannel pipetting. Serial dilutions (1 in 4) started at a final concentration of 10,000 nM. Final concentrations >10,000 nM were individually prepared from the 1mM stock solution. Nonspecific binding was defined by 100mM serotonin creatinine sulfate (Research Biochemicals) prepared fresh in incubation buffer at the time of each determination, and protected from light. in incubation buffer at the time of each determination, and protected from light. Reaction volumes were as follows: 200ml incubation buffer (50 mM tris, 0.5m M EDTA, 10mM MgCl₂), pH 7.4 at 22 0 C,100 ml test agent or serotonin (100mM) or buffer, 100 ml [3H]lysergic acid diethylamide (2nM final concentration) and100 ml membrane preparation (15 mg protein). The incubation was initiated by the addition of membrane homogenate and the plates vortexed. The plates were incubated, with protection from light, by shaking at 37 0 C for 60 min. The binding reaction was stopped by filtration. The samples were filtered under vacuum over 96 well glass fiber filters, presoaked in 0.3% PEI in 50 mM tris buffer (pH 7.4) for at least 1H and then washed 6x with 1 ml of cold 50 mM tris (4 0 C, pH 7.4) using a Packard Filtermate Harvester. The unifilter plates were dried overnight in a 37 ° C dry incubator. The unifilter bottoms were sealed and 35 ml of Packard MicroScint was added. The plates were allowed to equilibrate for 1h and were then sealed using a Packard TopSeal P with the Packard Plate Micromate 496. Plates were counted by liquid scintillation spectrometry. Each well was counted for 3 min. Compounds were initially assayed at 1000 and 100 nM. If a compound caused at least 80% inhibition of [³H]lysergic acid diethylamide binding at 1000 nM, it was further tested and a Ki determined. The range of concentrations chosen was so that the middle concentration would produce approximately 50% inhibition.

**Table1 shows the affinity for 5-HT₆ receptors of claimed compounds derived from the general formula in formula 2 above.**

| COMPOUND | R1 | R2 | R3 | R4 | R5 | Ki(nm) |
|---|---|---|---|---|---|---|
| A | Me | Me | 5-OMe | H | Me | 60 |
| B | Me | Me | 5-OMe | H | Et | 16 |
| C | Me | Me | 5-OMe | H | nPr | 185 |
| D | Me | Me | 5-OMe | H | Phenyl | 20 |

**Table 2 shows the results of affinity for 5-HT6 receptors of daimed and reference compounds derived from the general formula in formula 3 above.**

| COMPOUND | R1 and R2 | R4 | Ki(nm) |
|---|---|---|---|
| E (Reference) | Me | -CH2-phenyl | 136 |
| F (Reference) | H | -Ch2-phenyl | 302 |
| G | Me | H | 168 |
| H (Reference) | Me | -SO2-phenyl | 2 |

**Table 3 shows the results of affinity for 5-HT₆ receptors of reference compounds derived from the general formula in formula 4 above.**

| COMPOUND | X | Ki(nM) |
|---|---|---|
| I | O | 84 |
| J | H2 | 1030 |

### EXAMPLE 13

### Characterization of 5HT₆ Selectivity

Selected compounds were examined to determine their specificity of binding to the 5-HT₆ receptor. Compounds were examined at more than 30 receptor populations. Assays for the following receptors were performed as per the NIMH Psychoactive Drug Screening Program. The compounds failed to displace radioligand (i.e., <50% displacement) at a concentration of 10,000 nM at most receptors. Where more than 50% displacement was observed, Ki values were determined and the data are reported in the following table. It can be seen that the compounds are selective for 5-HT₆ receptors.

Table 4 shows the 5-selectivity of several compounds of the invention.

**TABLE 4 Ki, nM(SEM)**

| Receptor Population | COMPOUND A | COMPOUND B | COMPOUND D | CONTROL AND AGENT |
|---|---|---|---|---|
| NET | 6,380(3190) | >10,000 | >10,000 | Nortriptyline 6.3 (1.2) |
| SERT | >10,000 | >10,000 | 4,700(1550) | Fluoxetine 3.5(0.7) |
| h5-HT1A | 200(60) | 170(54) | 1,470(310) | WAY 1000,635 0.6 (1.5) |
| h5-HT1D | 250(180) | 290(700) | 6,225(70) | Ergotamine0. 8 (0.6) |
| h5-HT1E | 1,800(600) | 520(180) | >10,000 | Serotonin0.5 (.015) |
| r5-HT2A | >10,000 | >10,000 | 470(10) | Clozapine 9(1) |
| r5-HT2C | 4,020(640) | 1,810(490) | 675(180) | Clozapine 23(5) |
| h5-HT5A | 10,450(2195) | 4,620(650) | 5,160(930) | Ergotamine 22(3) |
| h5-HT7 | 145(34) | 300(60) | 155(35) | Clozapine 9(2) |
| h5-Ht6 | 60(13) | 16(4) | 20(5) | Clozapine 10(3) |

Compounds displayed Ki values of>10,000 nM at the following populations of receptors: histamine, NMDA, PCP, acetylcholine, opiate, and vasopressin receptors. Ki values were >10,000 nM for compounds A and B at hD1,rD2,rD3,rD4, and hD5 receptors and 10,000 nM for D at hD1,rD2, and rD4 receptors. Compound D produced 70% inhibition at rD3 and hD5 receptors. NET and SERT represent the norepinephrine and serotonin transporters. Ki values for all three compounds were >10,000 at the dopamine transporter.

### EXAMPLE 14

cAMP activation assays. Human 5-HT₆ receptors stably expressed in 293 HEK cells were grown in 24-well plates to near confluence and 18 h prior to the assay the medium was replaced with DMEM containing dialyzed 10% Fetal Calf Serum. For the assay, the medium was aspirated and replaced with fresh DMEM without serum and incubated with various concentrations of compounds of the invention in a total volume of 0.5 ml for 15 min. The assay was terminated by aspiration and the addition of 10% trichloroacetic acid (TCA). The TCA extract was used for cAMP determinations. (Data represent the mean of N=4 separate determinations). Results of cAMP activation by various compounds of the invention are shown in the attached Drawing of Figure 1.

## Claims

1. A compound having the formula: 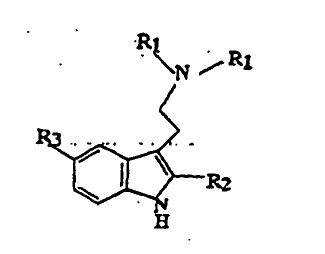
wherein,
R¹ is selected from the group consisting of hydrogen, methyl, ethyl or propyl and may be different at each location,
R² is selected from the group consisting of ethyl, propyl, and phenyl, and
R³ is selected from the group consisting of hydrogen, methyl, ethyl, propyl and methoxy or a pharmaceutically acceptable salt thereof.

2. A compound or salt according to claim 1 wherein R¹ is methyl, R² is selected from the group consisting of ethyl and phenyl, and R³ is methoxy.

3. A compound or salt according to claim 1 selected from the group consisting of 2-ethyl-5-methoxy-N,N-dimethyltryptamine and 5-methoxy-2-phenyl-N,N-dimethyltryptamine.

4. A pharmaceutical composition comprising a pharmaceutically acceptable carrier and a compound or salt according to claim 3.

5. A compound according to claim 1 having the formula: 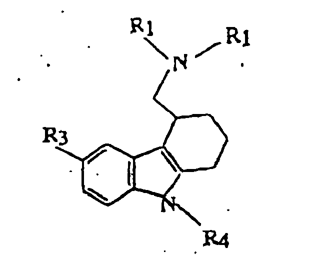
wherein,
R¹ is selected from the group consisting of hydrogen, and methyl, ethyl or propyl and may be the same or different at each site; and
R⁴ is selected from the group consisting of hydrogen,methyl, ethyl or propyl; and
R³ is selected from the group consisting of hydrogen, methyl, ethyl, propyl and methoxy, or pharmaceutically acceptable salt thereof.

6. A compound or salt according to one of the claims 1 to 5 further comprising a radiolabel.

7. A use of a compound selected from the group consisting of: 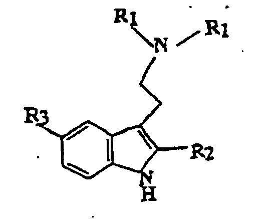 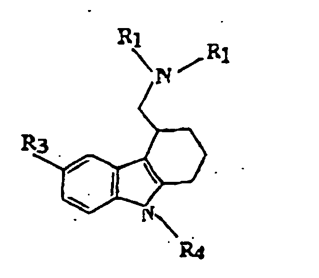
wherein
R¹ is selected from the group consisting of hydrogen and methyl, ethyl or propyl and may be the same or different at each site;
R² is selected from the group consisting of ethyl, propyl, phenyl and alkaphenyl;
R³ is selected from the group consisting of hydrogen, methyl, ethyl, propyl and methoxy and
R⁴ is selected from the group consisting of hydrogen, ethyl or propyl,
or pharmaceutically acceptable salt thereof and a pharmaceutically acceptable carrier for the preparation of a therapeutically effective composition for treating a condition mediated by the 5-HT₆ receptor.

8. The use of claim 7 further comprising a radiolabel.

9. A compound or a composition as claimed in any one of claims 1 to 8 **characterised in that** the compound is in the form of a pharmaceutically acceptable acid addition salt.

## Patentansprüche

1. Verbindung mit der Formel: 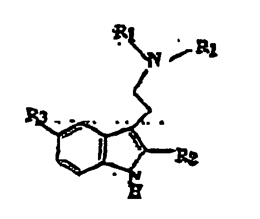
wobei
R¹ aus der Gruppe bestehend aus Wasserstoff, Methyl, Ethyl oder Propyl ausgewählt ist und an jeder Stelle unterschiedlich sein kann,
R² aus der Gruppe bestehend aus Ethyl, Propyl und Phenyl ausgewählt ist, und
R³ aus der Gruppe bestehend aus Wasserstoff, Methyl, Ethyl, Propyl und Methoxy ausgewählt ist,
oder ein pharmazeutisch verträgliches Salz davon.

2. Verbindung oder Salz nach Anspruch 1, wobei R¹ Methyl ist, R² aus der Gruppe bestehend aus Ethyl und Phenyl ausgewählt ist und R³ Methoxy ist.

3. Verbindung oder Salz nach Anspruch 1, ausgewählt aus der Gruppe bestehend aus 2-Ethyl-5-methoxy-N,N-dimethyltryptamin und 5-Methoxy-2-phenyl-N,N-dimethyltryptamin.

4. Pharmazeutische Zusammensetzung umfassend einen pharmazeutisch verträglichen Träger und eine Verbindung oder ein Salz gemäß Anspruch 3.

5. Verbindung nach Anspruch 1 mit der Formel: 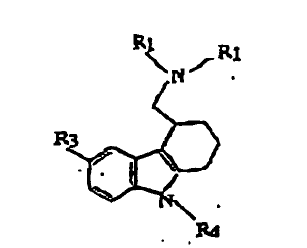
wobei
R¹ aus der Gruppe bestehend aus Wasserstoff und Methyl, Ethyl oder Propyl ausgewählt ist und an jeder Stelle gleich oder unterschiedlich sein kann; und
R⁴ aus der Gruppe bestehend aus Wasserstoff, Methyl, Ethyl oder Propyl ausgewählt ist; und
R³ aus der Gruppe bestehend aus Wasserstoff, Methyl, Ethyl, Propyl und Methoxy ausgewählt ist,
oder ein pharmazeutisch verträgliches Salz davon.

6. Verbindung oder Salz nach einem der Ansprüche 1 bis 5 ferner umfassend eine Radiomarkierung.

7. Verwendung einer Verbindung ausgewählt aus der Gruppe bestehend aus: 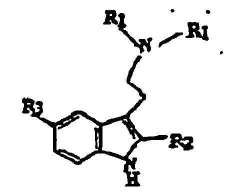 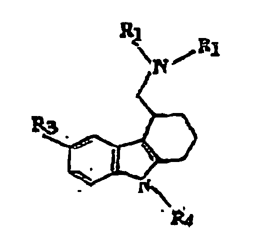
wobei
R¹ aus der Gruppe bestehend aus Wasserstoff und Methyl, Ethyl oder Propyl ausgewählt ist und an jeder Stelle gleich oder unterschiedlich sein kann;
R² aus der Gruppe bestehend aus Ethyl, Propyl, Phenyl und Alkaphenyl ausgewählt ist;
R³ aus der Gruppe bestehend aus Wasserstoff, Methyl, Ethyl, Propyl und Methoxy ausgewählt ist, und
R⁴ aus der Gruppe bestehend aus Wasserstoff, Ethyl oder Propyl ausgewählt ist,
oder ein pharmazeutisch verträgliches Salz davon und einen pharmazeutisch verträglichen Träger zur Herstellung einer therapeutisch wirksamen Zusammensetzung für die Behandlung eines durch den 5-HT₆-Rezeptor-vermittelten Zustandes.

8. Verwendung nach Anspruch 7, ferner umfassend eine Radiomarkierung.

9. Verbindung oder Zusammensetzung nach einem der Ansprüche 1 bis 8, **dadurch gekennzeichnet, dass** die Verbindung in Form eines pharmazeutisch verträglichen Säureadditionssalzes vorliegt

## Revendications

1. Composé répondant à la formule : 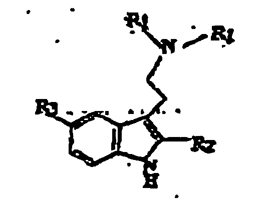
dans laquelle,
R¹ est choisi dans le groupe constitué par un atome d'hydrogène, un groupe méthyle, éthyle ou propyle et peut être différent à chaque emplacement,
R² est choisi dans le groupe constitué par les groupes éthyle, propyle et phényle, et
R³ est choisi dans le groupe constitué par un atome d'hydrogène, les groupes méthyle, éthyle, propyle et méthoxy,
ou sel phannaceutiquement acceptable de celui-ci.

2. Composé ou sel selon la revendication 1, dans lequel R¹ représente un groupe méthyle, R² est choisi dans le groupe constitué par les groupes éthyle et phényle, et R³ représente un groupe méthoxy.

3. Composé ou sel selon la revendication 1, choisi dans le groupe constitué par la 2-éthyl-5-méthoxy-N,N-diméthyltryptamine et la 5-méthoxy-2-phényl-N,N-diméthyltryptamine.

4. Composition pharmaceutique comprenant un véhicule pharmaceutiquement acceptable et un composé ou un sel selon la revendication 3.

5. Composé selon la revendication 1, répondant à la formule : 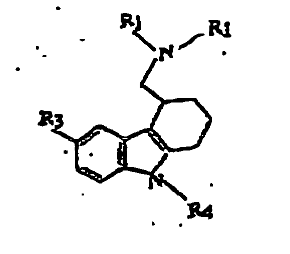
dans laquelle,
R¹ est choisi dans le groupe constitué par un atome d'hydrogène et un groupe méthyle, éthyle ou propyle, et peut être identique ou différent à chaque site ; et
R⁴ est choisi dans le groupe constitué par un atome d'hydrogène, un groupe méthyle, éthyle ou propyle ; et
R³ est choisi dans le groupe constitué par un atome d'hydrogène, les groupes méthyle, éthyle, propyle et méthoxy,
ou sel pharmaceutiquement acceptable de celui-ci.

6. Composé ou sel selon l'une quelconque des revendications 1 à 5, comprenant en outre un radio-marqueur.

7. Utilisation d'un composé choisi dans le groupe constitué par : 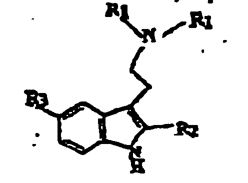 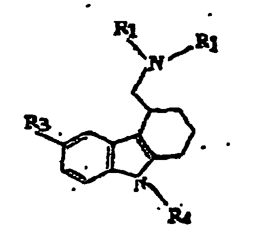
où R¹ est choisi dans le groupe constitué par un atome d'hydrogène et un groupe méthyle, éthyle ou propyle et peut être identique ou différent à chaque site ;
R² est choisi dans le groupe constitué par les groupes éthyle, propyle, phényle et alkaphényle ;
R³ est choisi dans le groupe constitué par un atome d'hydrogène, les groupes méthyle, éthyle, propyle et méthoxy et
R⁴ est choisi dans le groupe constitué par un atome d'hydrogène, un groupe éthyle ou propyle,
ou d'un sel pharmaceutiquement acceptable de celui-ci et d'un véhicule pharmaceutiquement acceptable pour la préparation d'une composition thérapeutiquement efficace pour le traitement d'un état à médiation par le récepteur 5-HT₆.

8. Utilisation selon la revendication 7, comprenant en outre un radio-marqueur.

9. Composé ou composition selon l'une quelconque des revendications 1 à 8, **caractérisé en ce que** le composé est sous la forme d'un sel d'addition avec un acide pharmaceutiquement acceptable.
